(19) **Europäisches Patentamt**

**European Patent Office**

**Office européen des brevets**

(11) **EP 1 531 871 B1**

(12) **EUROPEAN PATENT SPECIFICATION**

(45) Date of publication and mention
of the grant of the patent:
**09.08.2006 Bulletin 2006/32**

(51) Int Cl.:
***A61K 49/06*** (2006.01)

(21) Application number: **03790941.3**

(86) International application number:
**PCT/EP2003/009587**

(22) Date of filing: **29.08.2003**

(87) International publication number:
**WO 2004/019995 (11.03.2004 Gazette 2004/11)**

(54) **METHOD AND APPARATUS FOR PRODUCING CONTRAST AGENTS FOR MAGNETIC RESONANCE IMAGING**

VERFAHREN UND VORRICHTUNG ZUR HERSTELLUNG VON KONTRASTMITTELN FÜR MAGNETRESONANZBILDGEBUNG

PROCEDE ET DISPOSITIF POUR LA PRODUCTION D'AGENTS DE CONTRASTE EN IRM

(84) Designated Contracting States:
**AT BE BG CH CY CZ DE DK EE ES FI FR GB GR HU IE IT LI LU MC NL PT RO SE SI SK TR**

(30) Priority: **29.08.2002 GB 0219954**

(43) Date of publication of application:
**25.05.2005 Bulletin 2005/21**

(73) Proprietor: **GE Healthcare AS
0485 Oslo (NO)**

(72) Inventors:
• **GOLDMAN, Maurice
F-91140 Villebon sur Yvette (FR)**
• **AXELSSON, Oskar, GE Healthcare AS
N-0401 Oslo (SE)**
• **JOHANNESSON, Haukur,
Amersham Health (R & D) AB
S-S-205 12 Malmo (SE)**
• **ARDENKJAER-LARSEN, J.,
Amersham Health (R & D) AB
S-S-205 12 Malmo (SE)**

(74) Representative: **Franks, Barry Gerard et al
GE Healthcare Limited
Amersham Place
Little Chalfont,
Bucks. HP7 9NA (GB)**

(56) References cited:
**WO-A-00/71166          WO-A-99/24080**

• **JENS BARKEMEYER ET AL: "Heteronuclear Polarization Transfer Using Selective Pulses during Hydrogenation with Parahydrogen" JOURNAL OF MAGNETIC RESONANCE, no. 0109, - 1996 pages 129-132, XP002267375 series A 120**
• **JOHANNES NATTERER ET AL: "Parahydrogen induced polarization" PROGRESS IN NUCLEAR MAGNETIC RESONANCE SPECTROSCOPY, no. 31, - 1997 pages 293-315, XP002267376**
• **K. GOLMAN ET AL: "Parahydrogen-Induced Polarization in Imaging: Subsecond 13C Angiography" MAGNETIC RESONANCE IN MEDICINE, vol. 46, 2001, pages 1-5, XP002267377**
• **HELMUT SENGSTSCHMID ET AL: "A New Excitation Sequence to Observe the PASADENA Effect" JOURNAL OF MAGNETIC RESONANCE, - 1996 pages 249-257, XP002267378 series A 120**

**Description**

**Field of the invention**

The present invention relates to an apparatus and method for para-hydrogen induced hyperpolarization of a compound, and in particular for the preparation of a contrast agent for magnetic resonance imaging procedures.

**Background of the Invention**

Magnetic Resonance Imaging (MRI) is an important diagnostic technique. It is especially attractive since it is non-invasive and does not expose the patient to potentially harmful radiation such as X-rays or radiation from radioactive materials. Significant progress has recently been made in the quality of images as well as finding new applications of the technique. The progress relies on a rapid development of the digital image processing, the refined Nuclear Magnetic Resonance (NMR) techniques and the development of effective contrast agents (imaging agents). An emerging technique of particular interest involves Magnetic Resonance (MR) contrast agents based on the principle of pre-polarization of the nuclear spins, also called hyperpolarization

For the resonance phenomena, which is the basis of NMR and MRI, to occur, isotopes with non-zero nuclear spin have to be present. In addition, since NMR is not an extremely sensitive technique, a relatively high concentration and/or a high gyromagnetic ratio is needed, especially for imaging purposes. The use of a selected isotope in a contrast agent which is to be injected in a patient, puts further requirements on the selected isotope, for example regarding toxicity. A number of isotopes have the required spin properties, but only a few are considered interesting for the use in contrast agents, for example the carbon isotope $^{13}C$ and the nitrogen isotope $^{15}N$. The carbon isotope $^{13}C$ has many properties that would it make it useful as a functional part of an MRI contrast agent. An important feature is the long longitudinal relaxation time, $T_1$. The relaxation time needs to be long in order to have time, after the generation of the contrast agent, to inject the contrast agent into the patient and to allow the contrast agent to be transported to the organ that is to be studied. To make a useful MR-contrast agent of this kind, the signal strength has to be boosted significantly over the thermal equilibrium signal. Patent application WO 00/71166, by the same applicant, describes a process and an apparatus for increasing the polarization and hence the signal from a small organic molecule containing for example $^{13}C$. The signal was increased with a factor $10^4$. The process is referred to as Para-Hydrogen Induced Polarisation (PHIP) and may comprise the transferring of nuclear spin-order from para-hydrogen to spin polarization in non-zero spin nuclei in molecules, e.g. to $^{13}C$ or $^{15}N$ nuclei.

Hydrogen molecules exist in four different spin states. In one of the forms, characterised by antiparallel spins, the magnetic moments of the protons cancel. This form is called parahydrogen. The other three forms, with a net magnetic moment, are referred to as ortho-hydrogen. The para-hydrogen will not rotate at low temperature whereas the ortho-form must rotate with a high frequency at all temperatures because of quantum-mechanical symmetry requirements of the wave function. This indicates that at low temperature the para-form will have a significantly lower energy, and hence is the energetically favoured form. At temperatures below 20 K the equilibrium ratio of para- and ortho-hydrogen approaches 100:0, at 80 K the ratio is 48:52 and at room temperature approximately 1:3. The equilibration can be speeded up by the presence of a transition metal catalyst, e.g. $Fe_2O_3$. Para-hydrogen relaxes slowly (if no catalyst is present) at room temperature.

In WO 00/71166 it is described how to catalytically hydrogenate (with para-hydrogen) unsaturated compounds comprising non-zero spin nuclei such as $^{13}C$. The spin correlation of the protons from the para-hydrogen will be preserved during and after hydrogenation, and the influence on the spins of the $^{13}C$ nuclei breaks the symmetry of the spin system. The protons will now give an NMR-signal, but the non-equilibrium spin order is not sufficient to make the molecule useful for imaging purposes since it has an anti-phase behaviour that is not ideal for imaging. In the above cited applications, and further in "Parahydrogen-Induced Polarization in Imaging" by K. Golman et al., Magnetic Resonance in Medicine 46:1-5 (2001), a magnetic field cycling method is described for transforming the proton spin-order to carbon spin polarization. In a first step the external magnetic field is reduced (from the relatively high geomagnetic field) bringing the combined proton-carbon spin system into its strong coupling regime. In this regime the scalar coupling (J-coupling) strongly influences the evolution of the spin system. The reduction of the field should be fast, giving a diabatic (non-adiabatic) process. In a subsequent step the field strength is slowly increased (an adiabatic process). The field cycling will result in a substantial increase in the polarization of the spins of the $^{13}C$ nuclei, giving an in-phase NMR-signal, and increase the usefulness of the compound as a contrast agent for use in imaging procedures. However, the result of the field treatment will be dependent on the scalar coupling of the combined spin system and the properties of the magnetic field. In WO 00/71166 examples of field cycling schemes are described that give a substantial improvement in the image quality. To make the method even more attractive for use in medical and diagnostic applications it would be of high value to further increase the degree of polarisation of the carbon spins and to prolong the relaxation time of the nuclear spin system.

**Summary of the Invention**

The object of the present invention is to provide a method and an apparatus for producing MRI contrast agent with a high degree of polarisation of the imaging nuclei spins.

The object is achieved by the method as defined in claim 1, the apparatus as defined in claim 11, and by the computer program product as defined in claims 17 and 18.

The method for producing contrast agent according to the present invention comprises the steps of obtaining a solution in a solvent of a hydrogenatable, unsaturated substrate compound and a catalyst for the hydrogenation of a substrate compound, hydrogenating the substrate with hydrogen gas ($H_2$) enriched in para-hydrogen (p-$^1H_2$) to form a hydrogenated contrast agent and exposing the contrast agent to a oscillating magnetic field in combination with a stationary magnetic field for enhancing the contrasting effects of the contrast agent adapted for use in an MR application.

According to one embodiment of the present invention the oscillating magnetic field is oscillating with a frequency within the region of radio frequencies (around 10 Hz to several GHz) and preferably with a frequency in the interval 5 kHz to 500 MHz.

According to a further embodiment of the present invention the step of exposure to the oscillating magnetic field in combination with the stationary magnetic field is performed during the step of hydrogenation, wherein the step of exposure is performed for reducing the relaxation of the spin system of the contrast agent, whereby the contrasting effects of the contrast agent is enhanced.

According to a still further embodiment of the invention the step of exposure to the oscillating magnetic field in combination with the stationary magnetic field is performed after the step of hydrogenation, the step of exposure is performed for enhancing the degree of polarization of imaging nuclei of the contrast agent, whereby the contrasting effects of the contrast agent is enhanced. The oscillating field is applied in the form of a series of pulses with the frequency and angle varying and adapted for the contrast agent in use.

The apparatus for producing MR contrast agent according to the present invention comprises means for producing an oscillating magnetic field and means for producing a stationary magnetic field. The means for producing both the oscillating magnetic field and the stationary magnetic field may advantageously be of the same type as commonly used in NMR equipment.

One advantage afforded by the apparatus and method according to the present invention is that contrast agent can be produced that significantly improves the image quality and/or speeds up the process in an MRI application and/or improves the analysis performance in an MNR application.

A further advantage is that novel types of imaging, not possible, or very difficult to perform with prior art techniques, can be performed.

The advantages are achieved by the apparatus and method according to the present invention by providing a high degree of polarization of the imaging nuclei of the contrast agent, by that the process of producing contrast agent is fast, thereby reducing the problems with relaxation of the spins system of the contrast agent and by that the relaxation of the spins system is effectively reduced.

**Brief Description of the Drawings**

The invention will now be described in detail with reference to the drawing figures, in which

Fig. 1 is a flowchart illustrating the main steps of the method according to the invention;

Fig. 2 is a schematic drawing illustrating the apparatus according to the invention;

Fig. 3 is a schematic drawing illustrating the magnetic treatment unit of the apparatus according to the invention is;

Fig. 4 is a flowchart illustrating one embodiment of the method according to the invention;

Fig 5 is a flowchart illustrating one embodiment of the method according to the invention;

**Detailed Description of the invention**

Parts of apparatus and parts of the process described in WO 00/71166 are advantageously utilised also in the present invention. WO 00/71166 teaches that the hydrogenation reaction is preferably performed by mixing gaseous para-hydrogen (or ortho-deuterium) enriched hydrogen with a solution of an unsaturated compound and a hydrogenation catalyst.

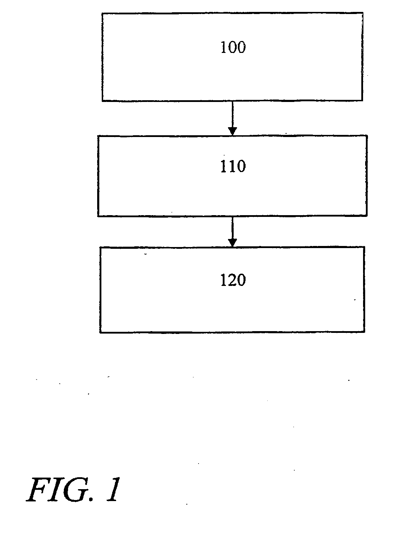

The main steps of the of the method according to the invention will be described with reference to the flowchart of FIG. 1 and comprises the following main steps:

100: obtaining a solution of a solvent, a hydrogenatable, unsaturated substrate compound and a catalyst for the hydrogenation of a substrate compound;

110: introducing the solution into a chamber containing hydrogen gas ($H_2$) enriched in parahydrogen (p- $^1H_2$) in order to hydrogenate the substrate to form a hydrogenated contrast agent.

The method according to the present invention introduces a main step of:

120: subjecting the contrast agent to an oscillating magnetic field in combination with a stationary magnetic field.;

The oscillations of the oscillating magnetic field should preferably be in the kHz-MHz-region. Such oscillating fields are commonly known as rf-fields, and the term rf-field will hereafter be used to denote an oscillating magnetic field with a frequency in the interval 5 kHz to 500 MHz. A frequency range of 10-500 kHz is preferred for many applications. The rf-field treatment enables spin-order to be transferred from protons in the freshly hydrogenated contrast agent into polarization of a nucleus within the same molecule with a slower relaxation, preferably a $^{13}C$ or $^{15}N$ nuclei. These nuclei will be referred to as imaging nuclei. In addition an rf-field may be used to reduce the relaxation rate of the spin systems during the hydrogenation-process, giving a higher polarization of the contrast agent. An apparatus and a method according to the present invention for providing the rf-field and stationary field during the production of the imaging agent is described below. The method and apparatus will be exemplified with contrast agent comprising carbon ($^{13}C$). This should be regarded as a non limiting example. Other isotopes could be used with slight modifications to the detailed steps of the method.

The hydrogenatable substrate used may be a material such as a para-hydrogenation substrate as discussed in WO99/24080. For *in vitro* or *in vivo* MR studies of biological or quasi-biological processes or synthetic polymer (e.g. peptide, polynucleic acid etc.) syntheses, the substrate is preferably hydrogenatable to form a molecule participating in such reactions, e.g. an amino acid, a nucleic acid, a receptor-binding molecule, etc., either a natural such molecule or an analog.

The solvent used in step 100 of the process of the invention may be any convenient material which serves as a solvent for the substrate and the hydrogenation catalyst. A number of possible solvents are discussed in WO99/24080. When the contrast agent is for use in *in vivo* MR investigations, the solvent is preferably physiologically tolerable. Water is a preferred choice of solvent, used in combination with a water-soluble catalyst. If other solvents that are not physiologically tolerable are used, the solvents has to be removed before use in a patient, for example by vacuum-spray. Other rapid solvent removal techniques, e.g. affinity techniques, may however be used. The solvent is preferably used at or near the minimum quantities required to maintain substrate, catalyst and contrast agent in solution without experiencing viscosity problems during the hydrogenation reaction.

The hydrogenation catalyst is preferably a catalyst as discussed in WO99/24080, e.g. a metal complex, in particular a rhodium complex.

The enriched hydrogen, which may be pure $^1H_2$ or $^2H_2$, or a mixture of $^1H_2$ and $^2H_2$, may optionally contain other gases, but is preferably free from oxygen or other reactive or paramagnetic gases, may be prepared by cooling hydrogen, preferably to a temperature below 80 K, more preferably to below 50 K, even more preferably to below 30 K and especially preferably to below 22 K, and allowing the nuclear spin states to equilibrate, optionally in the presence of a solid phase equilibration promoter, e.g. $Fe_3O_4$, $Fe_2O_3$, activated charcoal, etc. The enriched hydrogen is then preferably removed from the equilibrator and optionally stored before use. A method of preparation and storage of enriched hydrogen is described in WO99/24080. A preferred and novel method of storage and equipment for that purpose has been developed by the inventors. The enriched hydrogen is transferred to and stored in gas cylinders made of inert material. Inert in this context should be understood as made up by material essentially free from paramagnetic materials (primarily iron) and other para-hydrogen relaxing compounds (e.g. palladium). Examples of inert materials suitable for the gas cylinders are aluminum and carbon fiber reinforced epoxy. The enriched hydrogen will decay slowly (in the order of 10% per week). Such a decay rate is acceptable for most applications, especially compared with the cost and handling problems of previous storing methods involving storing at cryogenic temperatures.

For the hydrogenation reaction, a reaction chamber is filled with enriched hydrogen optionally under pressure, preferably 5-20 bar, and the catalyst and substrate solution is introduced either as a thin jet, by spraying or by atomising, into this reactor. If desired, the solution may be produced by mixing separate solutions of catalyst and of substrate. To ensure proper mixing, a distributor or a plurality of spray nozzles may be used and the chamber contents may be mixed, e.g. by a mechanical stirrer or by appropriately shaping the chamber walls to promote turbulent mixing where there is a flow of reaction mixture in the chamber.

The process may be performed continuously with a flow reactor, e.g. a loop or tube reactor, or alternatively it may be a batch-wise process. Preferably there will be a continuous or pulsed flow of enriched hydrogen and solution into the reactor, a continuous or batch-wise removal of liquid solution from the base of the reactor, and a continuous or batch-wise venting of unreacted gas from the reactor. The enriched hydrogen and solution passing into the reactor are preferably temperature-controlled to ensure the gas/droplet phase in the reactor is at the desired temperature. This can be achieved by providing input lines with temperature sensors and heating and/or cooling jackets.

If a non-aqueous solution has been used the contrast agent is preferably mixed with water after the hydrogenation and the exposure to the magnetic fields. The water used is preferably sterile and also preferably essentially free of paramagnetic contaminants. The resultant aqueous solution is then preferably treated to remove the hydrogenation catalyst, e.g. by passage through an ion exchange column, preferably one free of paramagnetic contaminants. The water may be temperature-controlled as may be a mixing chamber where water and contrast agent solutions are mixed so as to ensure the aqueous solution enters the ion exchange column at the appropriate temperature. Strongly acidic, sodium ion charged ion exchange resins such as DOWEX 1x2-400 (Dow Chemicals) and Amberlite IR-120 (both available from Aldrich Chemicals) resins may conveniently be used for the removal of typical metal complex hydrogenation catalysts. For fast ion exchange, the resin is preferably cross-linked to only a low degree, e.g. a 2% divinyl benzene cross-linked sulphonated, sodium ion loaded polystyrene resin.

Removal of the non-aqueous solvent may then conveniently be effected by spray flash distillation e.g. by spraying the aqueous solution into a chamber, applying a vacuum, and driving the organic solvent free aqueous solution from the chamber using an inert, preferably non-paramagnetic gas, e.g. nitrogen. Indeed in general the flow of liquid components through the hydrogenation apparatus is preferably effected using applied nitrogen pressure, e.g. 2 to 10 bar.

The resulting aqueous contrast agent solution may be frozen and stored or may preferably be used directly in an MR imaging or spectroscopy procedure, optionally after dilution or addition of further solution components, e.g. pH modifiers, complexing agents, etc. Such direct use may for example involve continuous infusion or alternatively injection or infusion of one or more dose units. Bolus injection is particularly interesting.

The whole process from beginning of hydrogenation to the delivery of the finished contrast agent in for example a syringe may conveniently be effected in less than 100 seconds, indeed it is feasible to produce dosage units in less than 10 seconds, which is substantially less than $T_1$ for the potentially interesting imaging nuclei.

Preferably, the surfaces contacted by the contrast agent during the process of the invention are substantially free of paramagnetic materials, e.g. made of glasses as used for hyperpolarized $^3$He containment as discussed in WO99/17304 or gold or polymer, optionally deuterated. Surfaces contacting a non-aqueous solvent (e.g. acetone) should be acetone-resistant and valves may be magnetically controlled and provided with solvent resistant Teflon or silicone parts.

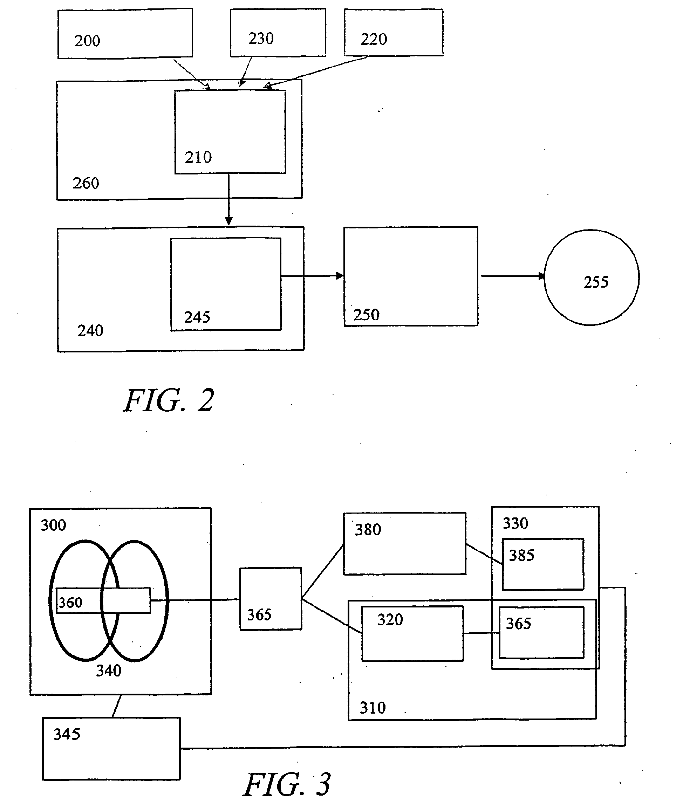

An apparatus suitable for producing contrast agent by the method according to the present invention will be described with reference to FIG. 2. Hydrogen ($^1$H$_2$) enriched in para-hydrogen is fed from the para-hydrogen source 200 into a reactor 210. A hydrogenation catalyst solution from a catalyst reservoir 220 and a hydrogenatable substrate solution from a substrate reservoir 230 are fed into the reactor 210. The liquid settling in reactor 210 is transferred to a magnetic treatment unit 240, which essentially comprises a compartment for the solution surrounded by a low field, two-channel NMR spectrometer, and thence to finishing unit 250 for cleanup, quality control and possible addition of additives and solvent removal. The finishing unit may comprise an ion exchange column and a solvent removal chamber equipped with a spray nozzle. After the passage through the finishing units the imaging agent is delivered to, for example, a syringe for injection in a patient. Alternatively the imaging agent is stored for later usage. The reactor 210, may in addition be provided with a initial magnetic treatment unit 260 adapted for producing a stationary magnetic field and an rf-field. As an alternative, the initial magnetic treatment unit 260 may be combined with the magnetic treatment unit 240 whereby all magnetic treatment will be performed within the reactor 210.

An embodiment of a magnetic treatment unit 240 in accordance with the present invention will be described with reference to the schematic block diagram of FIG. 3, in which the main parts of the NMR-unit are outlined. The same type of NMR-unit may be utilized as the initial magnetic treatment unit 260, or a combined magnetic treatment unit. The NMR unit comprises a coil system 300, a radio frequency synthesizer 310, an amplifier 320, and a computer 330. The coils of the coil system 300 are connected to power supplies 345, which preferably are connected to and controllable by the computer 330. The coil system comprises a resistive magnet that gives a main field of 0.1-100 mT, preferably 1-10 mT. This magnet may preferably be made as a Helmholz pair 340 with a large radius, around 30 cm, to give a high homogeneity over the sample space which preferably has a volume of 10-100 mL in the center of the coil system. The coil system 300 is preferably provided with three linear shim coils, x, y, and z (not shown) to allow for compensation of external field gradients, which primarily may be due to the presence of a nearby MRI system. The inhomogeneity of the Helmholtz pair will also be improved by the shims. Any other geometry (solenoidal) or magnet design (permanent magnet) compatible with the general design of the equipment can be used. Resistive magnets are suitable, due to the moderate field levels required. Superconducting magnets may also be used. Higher order shims can be added if there is need to place this equipment very close to the MRI magnet. When required, the magnet coils may, completely or in part, be wound from aluminum wire to reduce weight. Pairs of Hall plates (not shown) or other magnetic field detectors may also be present to allow for measurement of the field gradients in x, y and z directions so that the computer 330 can compensate for field disturbances automatically. The Hall sensors provide in addition the mean value of the magnetic field strength and is used to lock the field to the transmitter frequencies.

An NMR-coil 360 is used to produce the excitation signal. The excitation signal is conveniently generated digitally. The rf-coil 360 is connected via switching means 365 and a amplifier 320 to a digital waveform generator 375, the amplifier and the waveform generator forming the rf-synthesizer 310. The digital waveform generator can be a stand-alone instrument or a PC card. The desired frequency spectrum and phase relations are created arbitrarily within the limits of the time resolution of the instrument/device. The excitation signal from the waveform generator is amplified to create the desired excitation field strength in the coil. Generally, the pulses will be long at the low frequencies anticipated, and consequently the excitation power is low, typically a few watts.

The NMR coil 360 can be realized in a number of ways. The simplest is a solenoid, which has a high efficiency, and is a preferred geometry at low frequencies. The quality factor of the coil is maximized by the use of Litz wire. The coil may be tuned (double tuned) or non-tuned during excitation. Likewise, the coil can be tuned or non-tuned in reception mode. The switching means 365 can be a mechanical relay or passive circuitry.

The receiver-part of the spectrometer is convenient for controlling and optimizing the spectrometer performance, but is not crucial for the production of the contrast agent. It can also be used for polarization measurement. An important part of the receiver-chain is the low noise amplifier 380, which is needed for enabling optimal performance of the spectrometer. The amplifier should have a sufficiently large bandwidth and low noise (e.g. less than 3dB). In the case that the coil is tuned the bandwidth may be increased by negative feedback (active damping).

The amplified signal is digitalized directly and filtered to an appropriate bandwidth by an A/D converter 385, preferably provided as a PC card. Interpolation and decimation reduce the signal frequency and number of sampling points. A Fast Fourier Transform (FFT) is applied to the signal in order to calculate the NMR spectrum.

Preferably, most of the functions are integrated in a PC environment, in particular the frequency generation and sampling. The automatic shimming and field locking can also be handled from the PC.

A method according to the invention, advantageously utilizing the above described apparatus, for increasing the polarization of a contrast agent, will now be described. The method causes a transfer of proton spin order to a J-coupled long-$T_1$ nucleus by rf-pulse sequences. The pulses are applied to the contrast agent prior to injection into for example a patient. The method is generally applicable for use with a large variety of substrates, but the pulse sequence will be specific for the substrate and the stationary magnetic field, $B_0$. The method will be exemplified with a molecule containing only one none-zero spin nucleus, $^{13}$C with S=1/2, which is hydrogenated with para-hydrogen and preferably in aqueous solution. It should be noted that the method according to the present invention is not limited to this example. The method of using rf-pulse sequences for spin order transfer is applicable to any molecules with spin 1/2 hetero nucleus.

A prerequisite for the method is that the hydrogenation catalyst works by such a mechanism that the two hydrogen atoms from one molecule of para-hydrogen are delivered to one molecule of substrate to produce one molecule of product. These protons pairs are then produced in a singlet spin state, which corresponds to a high degree of order, and the rf-pulse sequence is intended to transfer this order to carbon polarization.

The hydrogenation is typically performed in the earth field, and the hydrogenation will occur at different times for the individual molecules. This will result in a system finally being in a statistical steady-state characterized by various populations of the energy states of the spin system, corresponding to the loss of the interstate initial correlations, and the subsequent loss of some of the initial order. The method according to the invention uses spin excitations by the way of radiofrequency pulses (rf-pulses) on either spin species, i.e. the frequency is adjusted to correspond to the Larmor-frequency of the nuclei. This is performed in a magnetic field sufficiently low such that the proton spins are in the so-called strong coupling regime, that is, the difference of the carbon-protons couplings and/or the difference of proton resonance frequencies due to different chemical shifts are not large compared with the inter-proton interactions. This has two important advantages. Firstly, there is no need to use a large magnetic field for the order-to-polarization step, and secondly the very existence of a strong inter-spin coupling is the root of the efficient manipulation of the spin state of the system.

In the embodiments of the present invention the notations defining angles of the applied fields refers to a rotating frame and are the notations commonly used in the discipline of NMR. The phrases "pulse on carbon", "pulse on hydrogen" etc should be understood as a rf-pulse with the frequency adapted to the nucleus and the stationary field as to correspond to the Larmor frequency of the nucleus.

Two alternative embodiments of the invention directly applicable in the case of the carbon nucleus having only two adjacent protons will be described. These prerequisites hold for e.g. 2,3-dideuterosuccinate-1-$^{13}$C. The case when more than two protons are coupled to the carbon nuclues is more complicated than the former one giving a much more complex structure of the quantum energy states. This requires parameters such as the delay times between pulses and the angles of the applied fields to be adjusted by optimization, either by computer simulation or by experiments.

The pulse sequence according to the first embodiment comprises two consecutive main parts: The first part is a preparation of the state of the system starting from the steady-state density matrix at the end of the hydrogenation, through 180°-pulses followed by evolution periods, and the second part is production of a net spin S (carbon) polarization by way of non-180° pulses (initially 90°) and evolution periods. In principle only the spins S are excited, but in practice one may apply 180° pulses on the spins $I$ (protons) simultaneously with 180° pulses on the spins $S$ to reduce the effects of field inhomogeneities.

The steady-state density matrix, $\sigma_{ss}$, after hydrogenation is given by the projection of the density matrix

$$\sigma = \tfrac{1}{8}\left(1 - 4\,\hat{\mathbf{I}}_1\hat{\mathbf{I}}_2\right)$$ on the eigen states of the Hamiltonian (rotating frame), $H = J_{12}\hat{I}_1\hat{I}_2 + J_{13}I_{1z}S_z + J_{23}I_{2z}S_z$, i.e.

$$\sigma_{ss} = \tfrac{1}{8}\left(1 - 4\left(I_{1z}I_{2z} + pS_z(I_{1z} - I_{2z}) + q(I_{1x}I_{2x} + I_{1y}I_{2y})\right)\right).$$ The coefficients $p$ and $q$ depend on the scalar couplings, $J_{ij}$, and determine the theoretical limit of the obtainable polarization.

The first main part consists of transforming the steady-state density matrix above to a state that as closely as possibly resembles $S_z(I_{1y}I_{2x} - I_{1x}I_{2y}) \equiv S_zK_y$, i.e. to optimize the (scalar) constant in front of this operator. This is achieved by a series of 180° pulses and subsequent evolution periods.

The second main part of the sequence starts with a $90_y(S)$ pulse that converts $S_zK_y$ into $S_xK_y$. The subsequent evolution transforms this state into $2S_xK_y \rightarrow \exp(-iHt)2S_xK_y\exp(iHt)$ which is a linear combination of $S_y$, $2K_yS_x$ and

$2K_zS_x$ (where $K_z \equiv \tfrac{1}{2}(I_{1z} - I_{2z})$). The coefficients in front of these terms oscillate, and the duration of the evolution will be chosen so that the term containing $S_y$ is maximized (at the same time $2K_zS_x$ vanishes). Then a $90_x(S)$ pulse is applied that produces a linear combination of $S_z$ and $2K_yS_x$. Another (identical) evolution time gives a linear combination of the terms $(S_z + CS_y)$ and $2K_yS_x$. A pulse of angle $\phi = \arctan(C)$, $\phi_x(S)$, will give the terms $S_z$ and $2K_yS_x$ (the last term is unaffected by a pulse on S around $O_x$). Neglecting relaxation a subsequent evolution will not affect $S_z$, whereas the term $2K_yS_x$ transforms as above. Repeating the above procedure, changing the angle $\phi$ accordingly, increases the polarization of carbon (i.e. $S_z$).

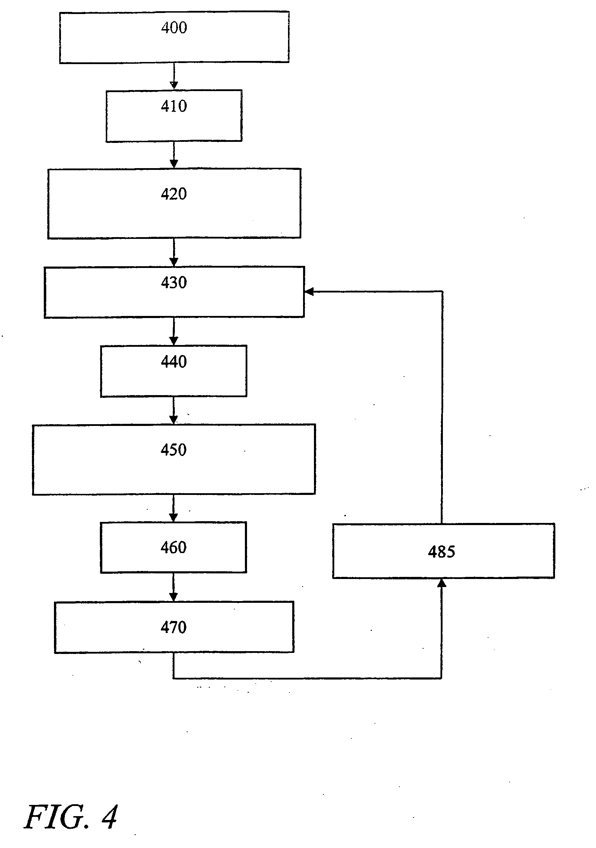

A realization of the first embodiment of the method according to the invention will be described with reference to the flowchart of FIG. 4. The realization of the first embodiment comprises the steps of:

400: Applying a stationary magnetic field $B_0$.

410: Hydrogenation of the substrate with para-hydrogen in the presence of the magnetic field $B_0$.

420: Applying a series of $180°_x$ pulses followed by delays $t_i$ on carbon. The delays after each pulse will typically differ. The delays $t_i$ are typically in the order of 1-100 ms. The number of repetitions are typically one or two but may be more. The purpose of this step is to bring the system into a state consisting of a zero quantum coherence involving the two protons and the carbon. The determination of delays and number of repetitions can be made analytically (if having one carbon and two protons) or by numerical methods (more spins) and involves maximizing the term $S_z(I_{1y}I_{2x} - I_{1x}I_{2y})$, in which S refers to the spin operator of carbon and $I_1$ and $I_2$ refer to the spin operators of the two protons. Step 420 corresponds to the first main part of the embodiment.

430: Applying a $90°_y$ pulse on carbon.

440: Waiting for $t/2$ s.

450: Optionally applying simultaneous $180°_x$ pulses on hydrogen and carbon in order to compensate for the effect of field inhomogeneities (spin echo pulses).

460: Optionally waiting for $t/2$ s.

470: Applying a pulse $\phi_x$ on carbon, the angle $\phi_x$ may be different in each step and as previously described determined by analytical calculation or by numerical simulation.

480: Repeating steps 440 to 470 $m$ times. Steps 440-470 gives a progressive build up of carbon polarization in the direction of the external field axis. The number of repetitions, $m$, is typically 1-5. The steps 430-480 corresponds to the second main part of the embodiment.

The first embodiment is characterized in that the pulses substantially effecting the polarization are applied only to the carbon nucleus, i.e. with a frequency corresponding to the Larmor frequency of carbon. The step involving pulses on both carbon and hydrogen is only taken to compensate for experimental imperfections. Depending on the molecule, a carbon spin polarization between 50% and 85% is typically achieved. The total duration of the polarization process is about one to several hundred milliseconds. Thus, the method is fast which can be crucial because of the loss of polarization due to spin relaxation.

The second embodiment comprises three consecutive main parts. The first main part transforms the steady-state density matrix (see above) to a state that as closely as possibly resembles

$$\frac{1}{8}\left(1 - 4\left(I_{1z}I_{2z} - I_{1x}I_{2x} - I_{1y}I_{2y}\right)\right) \equiv \frac{1}{8}\left(1 - 4\left(I_{1z}I_{2z} - K_x\right)\right),$$ i.e. optimizes the (scalar) constant in front of the operator $K_x$ (optimally equal to one). This is accomplished by a series of 180° pulses and subsequent evolution periods of equal duration. At the end of this part the density matrix is proportional to (disregarding small terms and the unimportant constant term) $I_{1z}I_{2z} + cK_x$ (c being close to unity). The second main part starts with a $90_y(I)$ pulse that produces the term $(I_{1x}I_{2x} - I_{1y}I_{2y})$. A subsequent evolution period gives the term $S_z(I_{1y}I_{2x} + I_{1x}I_{2y})$. The third main part starts with a $90_y$ (S) pulse transforming $S_z \rightarrow S_x$ and a $90_\phi(I)$ pulse, with a suitable phase, $\phi$, that transforms $(I_{1y}I_{2x} + I_{1x}I_{2y})$ into $(I_{1z}I_{2z} - I_{1y}I_{2y})$. The useful part of the density operator is now $2S_xI_{1z}I_{2z}$. An evolution period produces the useful part $S_x$ (neglecting terms that are of no interest) which is finally transformed to $S_z$ by a $90_y(S)$ pulse.

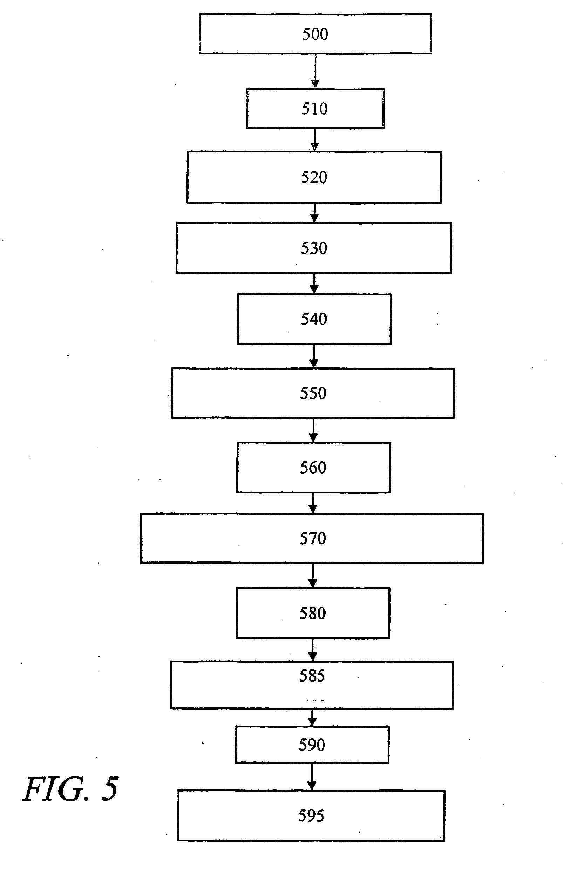

A realization of the second embodiment of the method according to the invention will be described with reference to the flowchart of FIG. 5. The realization of the second embodiment comprises the steps of:

500: Applying a stationary magnetic field $B_0$.

510: Hydrogenation of the substrate with para-hydrogen in the presence of the magnetic field $B_0$.

520: Applying a series of $180°_x$ pulses followed by delays $t_1$ on carbon. The number of repetitions are typically one or two but may be more. The purpose of this step is to bring the system into a state consisting of a two proton zero quantum coherence. The determination of delays and number of repetitions can be made analytically (one carbon, two protons) or by numerical methods (more spins) and involves maximizing the factor $(I_{1x}I_{2x} - I_{1y}I_{2y})$. Step 520 corresponds to the first main part of the second embodiment.

530: Applying a $90°_y$ pulse on hydrogen, which results in a two-proton-double quantum coherence.

540: Waiting for $t_2/2$ s. During this time the two-proton-double quantum coherence transforms into a three-spin coherence involving the carbon spin. Steps 530 and 540 corresponds to the second main part of the second embodiment.

550: Optionally applying simultaneous $180°_x$ pulses on hydrogen and carbon in order to compensate for the effect of field inhomogeneities (spin echo pulses).

560: Optionally waiting for $t_2/2$ s.

570: Applying simultaneous $90°_y$ and $90°_\phi$ pulses on carbon and hydrogen, respectively, for producing a transverse carbon polarization.

580: Waiting for $t_3/2$ s.

585: Applying simultaneous $180°_x$ pulses on carbon and hydrogen.

590: Waiting for $t_3/2$ s.

595: Applying a $-90°_y$ pulse on carbon, for transforming the transverse carbon polarization into a longitudinal polarization, which is parallel to the applied field. Steps 570-595 corresponds to the third main part of the second embodiment.

It should be noted that in the second embodiment, contrary to the first embodiment, rf-pulses directly effecting the polarization are applied to both the carbon and hydrogen nuclei. The polarizations achievable by the second embodiment are typically between 60% and 98%, depending on the molecule. For most instances, they are larger than in the first embodiment, but the pulse sequence is in most cases more complex and the duration of the polarization sequence is in general longer. Hence, the loss due to spin relaxation during the increased process duration has to be considered. Which of the embodiments to use under practical conditions is thus decided based on these factors and on the capability of the equipment. The decision is typically "case by case". Implementation examples and simulated results are enclosed in Appendix A. As appreciated by the one skilled in NMR and MRI, the tools and practices utilized in determining the pulse sequences in NMR and MRI applications can also be used as a guidance for determination of the pulse sequences used for producing contrast agent according to the present invention.

It is conceivable to use pulses that are modulated in amplitude, phase or frequency or any combination of those. Alternatively, the individual pulses can be substituted with composite pulses (i.e. sequences of phase-shifted rf pulses). This provides better phase-control over a wider bandwidth. This gives better results in practice and relaxes the demands on precision of the hardware. Examples of composite pulses can be found in: "Band-Selective Radiofrequency Pulses", Geen, H. Freeman, R. *J. Magn. Res.* **93**,93-141(1991), "Composite Pulses" Levitt, M. *Progress in NMR spectroscopy*, **18**, 61-122 (1986), "Composite Pulses without Phase Distortion" Tycko, R. *J. Magn. Res.* **61**, 90-101 (1985) and "Broadband, Narrowband, and Passband Composite Pulses for Use in Advanced NMR Experiments" Wimperis, S. *J. Magn. Res. Ser A* **109**, 221-231 (1994).

In both the described embodiments the delay times, $t_i$, are typically in the order of 1-100 ms. The amplitude of the rf-fields is typically 1-100 $\mu$T, the duration of the pulses follows from the rf-amplitude and the frequency adapted to the Larmor frequency of the target nucleus. The stationary magnetic field $B_0$, applied in step 400 and 500 respectively, is preferably 0.1-100 mT, and more preferably 1-10 mT.

The method according to the invention will facilitate the production of highly polarized contrast agent. The properties of the contrast agent may further be improved if the relaxation rate of the spin system is reduced. One way of reducing the relaxation rate of the spin system, i.e. extending the useful lifetime of the final contrast agent, is by decoupling of the former para-hydrogen protons during the process of hydrogenation. The apparatus and the method according to the invention of subjecting the contrast agent to rf-pulses, may advantageously be used also to reduce the relaxation of the spin system.

During the process of hydrogenation a substantial relaxation of the spin system occurs. The relaxation takes place both when the para-hydrogen is attached to the catalyst in the intermediate state and later, when it is transferred to the molecules of the contrast agent. The relaxation results in a reduced PHIP effect and impairs the performance of the contrast agent. During the hydrogenation different molecules will be hydrogenated at different times and the former para-hydrogen protons will no longer be in the singlet state: Intramolecular dipole-dipole coupling will cause relaxation of the spin system. The dipole-dipole interaction contribution to the relaxation can be significantly reduced by forcing the protons to remain in the singlet state. From a quantum mechanical consideration of the spin system it can be realized that, by letting the process of hydrogenation take place in the presence of both a rf-field and a static magnetic field, the protons can be forced to remain in their singlet state. Hence the relaxation can be significantly reduced.

In order to utilize the method of exposure to rf-field and magnetic field during hydrogenation the reactor 210 has to be equipped with means for producing the fields. In addition the material of the reactor 210 has to be transparent both to the rf irradiation and the static magnetic field. Preferably a non-conducting, i.e non-metallic material is used, for example glass-fibre reinforced epoxy. This material is also capable of withstanding the substantial pressure used during the process. The external static magnetic field and the rf-fields can preferably be provided by a magnet system similar to that of the magnetic treatment unit 240 described above. Alternatively the external magnetic field may be provided by a solenoid coil. The required field strength is in the order of 1 mT and the requirement of homogeneity is fairly moderate - inhomogeneities of up to $\pm$6% are acceptable. The rf-field may preferably be provided by crossed orthogonal saddle coils, which can produce circularly polarized rf-fields as well as more complex decoupling sequences. The frequency of the rf-field should correspond to the Larmor frequency of the protons, e.g. 42.6 kHz if the external field is 1 mT $\nu = \dfrac{\gamma B_0}{2\pi}$ , where $\nu$ is frequency of the rf-field, $\gamma$ the gyromagnetic ratio and $B_0$ the magnetic field). The intensity of the rf-field should preferably be optimized for each case. A suitable rf-field amplitude will typically be approximately 50 $\mu$T, with an acceptable inhomogeneity of the order of 10 $\mu$T. The generation and control of the required fields are well-known in the area of NMR and the chosen implementation can be modified in many ways as appreciated by those skilled in the art. All the magnetic field treatments are preferably done with the same magnet system and in the same chamber. Thus, in this preferred embodiment of the apparatus, the reactor 210 and the magnetic treatment unit 240 are combined into one unit.

The embodiments of the method according to the invention described above may be modified to also include the magnetic treatment for reducing the relaxation by letting the step of hydrogenation (step 400, 500) to be performed in the presence of a combined external magnetic field and an rf-field. The step in this preferred embodiment being:

400, 500:   Hydrogenation of the substrate with para-hydrogen, performed in the presence of an external stationary magnetic field and an external rf-field.

The method of exposing the contrast agent to a combined external magnetic field and an rf-field during the hydrogenation may be combined also with methods of spin order transfer other than the here described method utilizing series of rf-pulses. For example, the method of using field cycling schemes described in WO 00/71166, may be improved if a combined external magnetic field and an rf-field is present during the hydrogenation.

As the magnetic treatment unit 240 in a preferred embodiment of the invention has all the facilities of a two-channel NMR spectrometer, it can be advantageously used for characterizing and checking the quality of the produced contrast agent. The ability of characterizing, for example measuring the degree of polarization in the produced contrast agent, is valuable in developing and fine-tuning the pulse sequences of the above-described embodiments. This is obviously most useful in the cases when the parameters of the pulse sequences have to be established from experiments, but also if the parameters are determined analytically or by computerized optimization, the degree of polarization may be increased by fine-tuning parameters, for example the delay times, experimentally.

The method of the invention is preferably implemented by means of a computer program product comprising the software code means for performing the steps of the method by controlling parts of the apparatus according to the invention. The computer program product is typically executed on the computer 330. The computer program is loaded directly or from a computer usable medium, such as a floppy disc, a CD, the Internet etc.

From the invention thus described, it will be obvious that the invention may be varied in many ways. Such variations are not to be regarded as a departure from the inventive concept, and all such modifications as would be obvious to one skilled in the art are intended for inclusion within the scope of the following claims.

**Appendix A**

Exemplary pulse sequences:

Pulses given in degrees with phase as subscript, delays as time t with index as subscript. Sequence 1-2 corresponds to the first embodiment of the invention, sequence 3 to the second embodiment and sequence 4 is a special case related to double hydrogenation.

Sequence 1a:

$^{13}C \quad 180_x\text{-}t_1\text{-}180_x\text{-} t_2\text{-}90_y\text{-}t_3/2\text{-}180_x\text{-}t_3/2\text{-} \phi1_x$

$^{1}H \quad \text{-----------------------}180_x$

Sequence 1b:

$^{13}C \quad 180_x\text{-}t_1\text{-}180_x\text{-} t_2\text{-}90_y\text{-}t_3/2\text{-}180_x\text{-}t_3/2\text{-} \phi1_x\text{-} t_3/2\text{-}180_x - t_3/2\text{-} \phi2_x$

$^{1}H \quad \text{-------------------}180_x\text{-}t_3\text{-----------}180_x$

Sequence 1c:

$^{13}C \quad 180_x\text{-}t_1\text{-}180_x\text{-} t_2\text{-}90_y\text{-}t_3/2\text{-}180_x\text{-}t_3/2\text{-} \phi1_x\text{-} t_3/2\text{-}180_x - t_3/2\text{-} \phi2_x\text{-} t_3/2\text{-}180_x - t_3/2\text{-} \phi3_x$

$^{1}H \quad \text{-----------------}180_x\text{-}t_3\text{----------}180_x\text{-}t_3\text{---------------}180_x$

Sequence 1d:

$^{13}C \quad 180_x\text{-}t_1\text{-}180_x\text{-} t_2\text{-}90_y\text{-}t_3/2\text{-}180_x\text{-}t_3/2\text{-} \phi1_x\text{-} t_3/2\text{-}180_x - t_3/2\text{-} \phi2_x\text{-} t_3/2\text{-}180_x - t_3/2\text{-} \phi3_x\text{-} t_3/2\text{-}180_x - t_3/2\text{-} \phi4_x$

$^{1}H \quad \text{-----------------}180_x\text{-}t_3\text{----------}180_x\text{-}t_3\text{-------------} 180_x\text{-}t_3\text{------------} 180_x$

### Sequence 2a:

$^{13}$C     $180_x$-$t_3$-$180_x$-$t_1$-$180_x$- $t_2$-$90_y$-$t_3/2$-$180_x$-$t_3/2$- $\phi 1_x$

$^1$H     ---------------------------------$180_x$

### Sequence 2b:

$^{13}$C     $180_x$-$t_3$-$180_x$-$t_1$-$180_x$- $t_2$-$90_y$-$t_3/2$-$180_x$-$t_3/2$- $\phi 1_x$- $t_3/2$-$180_x$ - $t_3/2$- $\phi 2_x$

$^1$H     -----------------------------$180_x$-$t_3$-----------$180_x$

### Sequence 2c:

$^{13}$C     $180_x$-$t_3$-$180_x$-$t_1$-$180_x$- $t_2$-$90_y$-$t_3/2$-$180_x$-$t_3/2$- $\phi 1_x$- $t_3/2$-$180_x$ - $t_3/2$- $\phi 2_x$- $t_3/2$ -$180_x$ - $t_3/2$- $\phi 3_x$

$^1$H     -----------------------------$180_x$-$t_3$-----------$180_x$-$t_3$---------------$180_x$

### Sequence 3:

$^{13}$C     $(180_x$-$t_1)_n$- $t_2/2$------$180_x$-$t_2/2$-$90_y$-$t_3/2$-$180_x$- $t_3/2$-$90_y$

$^1$H     ----------$90_y$-$t_2/2$-$180_x$-$t_2/2$-$90_\phi$ -$t_3/2$-$180_x$

### Sequence 4:

$^{13}$C     $180_x$-$t_1$-$90_y$-$t_2/2$-$180_x$-$t_2/2$-$90_x$

$^1$H     --------------$180_x$

**Examples of predictions**

Contrast agent: Maleic acid

$J_{HH}$=10.65 Hz, $J_{HIC}$=15. 5 Hz, $J_{H2C}$=0.5 Hz

Pulse Sequence 1a, $B_0$=5 mT, $t_1$= 20.76 ms, $t_2$=33.91 ms, $t_3$=38.39 ms, $\phi 1$=90°:

Polarization P= 76.98 % , Total duration = 93.06 ms.

Pulse Sequence 1b, $B_0$=5 mT, $t_1$= 20.76 ms, $t_2$=33.91 ms, $t_3$=38.39 ms, $\phi 1$=90°, $\phi 2$=18.62°:

Polarization P= -81.23%, Total duration =131.45 ms.

Pulse Sequence 1c, $B_0$=5 mT, $t_1$= 20.76 ms, $t_2$=33.91 ms, $t_3$=38.39 ms, $\phi 1$=90°, $\phi 2$=-18.62°, $\phi 3$=6.14°:

Polarization P= 81.7%, Total duration = 169.83 ms.

Pulse Sequence 3, $B_0$=5 mT, n=2, $t_1$=38.4 ms, $t_2$=31.25 ms; $t_3$=69 ms:

Polarization P= 85.4% , Total duration = 177.0 ms.

Contrast agent: succinic acid (2,3-D$_2$)

$J_{HHvic}$=7 Hz, $J_{H1C}$=7 Hz, $J_{H2C}$= 0 Hz, $\Delta\delta H$= 0 ppm

Pulse Sequence 1a, B$_0$=5 mT, t$_1$= 44.09 ms, t$_2$=46.64 ms, t$_3$=63.89 ms, $\phi$1=90°:

Polarization P= 71.55 % , Total duration = 154.62 ms.

Pulse Sequence 1b, B$_0$=5 mT, t$_1$= 44.09 ms, t$_2$=46.64 ms, t$_3$=63.89 ms, $\phi$1=90°, $\phi$2=-30.96°:

Polarization P= -83.45% , Total duration = 218.51 ms.

Pulse Sequence 1c, B$_0$=5 mT, t$_1$= 44.09 ms, t$_2$=46.64 ms, t$_3$=63.89 ms, $\phi$1=90°, $\phi$2=30.96°, $\phi$3=17.16°:

Polarization P= 87.33% , Total duration = 282.4 ms.

Pulse Sequence 1d, B$_0$=5 mT, t$_1$= 44.09 ms, t$_2$=46.64 ms, t$_3$=63.89 ms, $\phi$1=90°, $\phi$2=30.96°, $\phi$3=17.16°, $\phi$3= -10.04°:

Polarization P= -88.69% , Total duration = 346.29 ms.

Pulse Sequence 3, B$_0$=5 mT, n=3, t$_1$=63.9 ms, t$_2$=71.4 ms, t3=135.4 ms:

Polarization P= 93.2%, Total duration = 398 ms.

Contrast agent: O-Acetyl lactic acid (3,3-D$_2$)

$J_{HH}$=7.1 Hz, $J_{H1C}$=7.3 Hz, $J_{H2C}$=2.4 Hz, $\Delta\delta H$= 3.2 ppm

Pulse Sequence 2a, B$_0$=5 mT, t$_1$= 40.07 ms, t$_2$=58.12 ms, t$_3$=66.57 ms, $\phi$1=90°:

Polarization P= 57.6 % , Total duration = 231.33 ms.

Pulse Sequence 2b, B$_0$=5 mT, t$_1$= 40.07 ms, t$_2$=58.12 ms, t$_3$=66.57 ms, $\phi$1=90°, $\phi$2=-38.2°:

Polarization P= -72.06% , Total duration = 297.9 ms.

Pulse Sequence 2c, B$_0$=5 mT, t$_1$= 40.07 ms, t$_2$=58.12 ms, t$_3$=66.57 ms, $\phi$1=90°, $\phi$2=-38.2°, $\phi$3=25.96°:

Polarization P= 78.80% , Total duration = 364.47 ms.

Pulse Sequence 3, B$_0$=5 mT, n=4, t$_1$=63.9 ms, t$_2$=71.4 ms, t3=135.4 ms:

Polarization P= 93.07% , Total duration = 424.7 ms.

Special case:Succinic acid, produced from acetylene dicarboxylic acid by two parahydrogen reactions in rapid sequence:

$J_{HHvic}$=7 Hz, $J_{HHgem}$=14 Hz, $J_{HIC}$=7 Hz, $J_{H2C}$= 0 Hz, $\Delta\delta H$= 0 ppm

Pulse sequence 4, t$_1$=28.43 ms, t$_2$=56.9 ms:

Polarization P=57%, Total duration =85.3 ms.

**Claims**

**1.** A method for producing MR contrast agent, the method comprising the steps of:

- *obtaining* (100) a solution in a solvent of a hydrogenatable, unsaturated substrate compound and a catalyst for the hydrogenation of a substrate compound, wherein the substrate compound comprises imaging nuclei;
- *hydrogenating* (110) the substrate with hydrogen gas (H$_2$) enriched in para-hydrogen (p-$^1$H$_2$) to form a hydro-

genated contrast agent;
- *exposing* (120) the contrast agent to a oscillating magnetic field in combination with a stationary magnetic field for enhancing the contrasting effects of the contrast agent adapted for use in an MR application.

2. The method according to claim 1 wherein the oscillating magnetic field is oscillating with a frequency within the region of radio frequencies (e.g from around 10 Hz to several GHz).

3. The method according to claim 1 wherein the oscillating magnetic field is oscillating with a frequency in the interval 5kHz to 500 MHz.

4. The method according to any of claim 2 wherein the step of exposure to the oscillating magnetic field in combination with the stationary magnetic field is performed during the step of hydrogenation, wherein the step of exposure is performed for reducing the relaxation of the spin system of the contrast agent, whereby the contrasting effects of the contrast agent is enhanced.

5. The method according to claim 2 wherein the step of exposure to the oscillating magnetic field in combination with the stationary magnetic field is to be performed after the step of hydrogenation, the step of exposure is performed for enhancing the degree of polarization of an imaging nuclei of the contrast agent, whereby the contrasting effects of the contrast agent is enhanced.

6. The method according to claim 5 wherein the step of exposure to the oscillating magnetic field in combination with the stationary magnetic field comprises exposing the contrast agent to at least one series of pulses of the oscillating magnetic field (rf-pulse).

7. The method according to claim 6 wherein the exposing step comprises:

   - *applying* (420) a first series of pulses of the Larmor frequency of the imaging nuclei of the hydrogenated contrast agent and delays between the pulses, the first series adapted to bring the system into a state consisting of a zero quantum coherence involving the protons and the imaging nuclei;
   - *applying* (430-480) a second series of pulses of the Larmor frequency of the imaging nuclei of the hydrogenated contrast agent and delays between the pulses, the second series adapted to give a progressive build up of carbon polarization in the direction of the external field axis.

8. The method according to claim 6 wherein the exposing step comprises:

   (a) -*applying* (420) a series of $180°_x$ pulses followed by delays($t_i$) on the imaging nuclei;
   (b) -*applying* (430) a $90°_y$ pulse on carbon;
   (c) -*waiting* (440) for $t/2$ s;
   (d) -Optionally *applying* (450) simultaneous $180°_x$ pulses on hydrogen and the imaging nuclei in order to compensate for the effect of field inhomogeneities;
   (e) -Optionally *waiting* (460) for $t/2$ s;
   (f) -*applying* (470) a pulse with an angle $\phi_x$ on the imaging nuclei;
   (g) -Optionally *repeating* steps c tofto produce a progressive build up of the imaging nuclei polarization in the direction of the external field axis, wherein the angle $\phi_x$ may be different in each repetition.

9. The method according to claim 6 wherein the exposing step comprises:

   - *applying* (520) a first series of pulses of the Larmor frequency of the imaging nuclei of the hydrogenated contrast agent and delays between the pulses, the first series adapted to bring the system into a state consisting of a zero quantum coherence involving the protons and the imaging nuclei;
   - *applying* (530-540) a second series of pulses and delays between the pulses comprising of pulses of the Larmor frequency of the imaging nuclei of the hydrogenated contrast agent alternated with pulses of the Larmor frequency of the hydrogen of the hydrogenated contrast agent, the second series adapted to transform a two-proton-double quantum coherence into a three-spin coherence involving the spins of the imaging nuclei;
   - *applying* (570) simultaneous $90°_y$ and $90°_\phi$ pulses on the imaging nuclei and hydrogen, respectively, adapted for producing a transverse polarization of the imaging nuclei.

10. The method according to claim 6 **wherein** the exposing step comprises:

- *applying* (520) a series of 180°$_x$ pulses followed by delays $t_1$ on the imaging nuclei;
- *applying* (530) a 90°$_y$ pulse on hydrogen;
- *waiting* (540) for $t_2/2$ *s;*
- optionally *applying* (550) simultaneous 180°$_x$ pulses on hydrogen and the imaging nuclei in order to compensate for the effect of field inhomogeneities;
- optionally *waiting* (560) for $t_2/2$ *s;*
- *applying* simultaneous (570) 90°$_y$ and 90°$_\phi$ pulses on the imaging nuclei and hydrogen;
- *waiting* (580) for $t_3/2$ s;
- *applying* (585) simultaneous 180°$_x$ pulses on the imaging nuclei and hydrogen;
- *waiting* (590) for $t_3/2$ *s;*
- *applying* (595) a -90°y pulse on carbon.

**11.** Method according to any of claims 7-10 wherein one or more of the radiofrequency pulses is either composite or modulated in amplitude, phase or frequency or any combination thereof.

**12.** Apparatus for producing MR contrast agent, the apparatus comprising a magnetic treatment unit (240) adapted for magnetic treatment of the contrast agent, **characterised in that** the magnetic treatment unit (240) comprises means for producing an oscillating magnetic field and means for producing a stationary magnetic field.

**13.** Apparatus according to claim 11 wherein said magnetic treatment unit (240) is combined with a hydrogenation reactor 210.

**14.** Apparatus according to claim 12 or 13 wherein said magnetic treatment unit (240) comprises essentially the magnetic system of a NMR spectrometer.

**15.** Apparatus according to claim 14 wherein said magnetic system of a NMR spectrometer additionally are used for analyzing the produced contrast agent with NMR spectroscopy.

**16.** Apparatus according to claim 12 or 13 wherein said magnetic treatment unit (240) comprises a Helmholtz pair (360) for producing the stationary magnetic field and a NMR coil (360) for producing the oscillating magnetic field.

**17.** A computer program product directly loadable into the internal memory of a processing means within a processing unit for controlling the method and apparatus for producing MR contrast agent, comprising the software code means adapted for controlling the steps of any of the claims 1 to 11.

**18.** A computer program product stored on a computer usable medium, comprising a readable program adapted for causing a processing means, in a processing unit for controlling the method and apparatus for producing MR contrast agent, to control an execution of the steps of any of the claims 1 to 11.

**Patentansprüche**

**1.** Verfahren zum Herstellen eines MR-Kontrastmittels, wobei das Verfahren die Schritte umfasst:

- Bereitstellen (100) einer Lösung einer hydrierbaren, ungesättigten Substratverbindung und eines Katalysators zur Hydrierung einer Substratverbindung in einem Lösungsmittel, wobei die Substratverbindung Bildgebungskerne umfasst;
- Hydrieren (110) des Substrats mit Wasserstoffgas ($H_2$), das in para-Wasserstoff (p-$^1H_2$) angereichert ist, um ein hydriertes Kontrastmittel zu bilden;
- Aussetzen (120) des Kontrastmittels einem oszillierenden Magnetfeld in Kombination mit einem stationären Magnetfeld zum Verstärken der Kontrastwirkung des Kontrastmittels, das zur Verwendung in einer MR-Anwendung angepasst ist.

**2.** Verfahren nach Anspruch 1, bei dem das oszillierende Magnetfeld mit einer Frequenz innerhalb des Bereichs von Radiofrequenzen oszilliert (z.B. von ungefähr 10 Hz bis mehreren GHz).

**3.** Verfahren nach Anspruch 1, bei dem das oszillierende Magnetfeld mit einer Frequenz im Intervall 5 kHz bis 500 MHz oszilliert.

**4.** Verfahren nach Anspruch 2, bei dem der Schritt des Aussetzens dem oszillierenden Magnetfeld in Kombination mit dem stationären Magnetfeld während des Schrittes der Hydrierung ausgeführt wird, wobei der Schritt des Aussetzens ausgeführt wird zum Verringern der Relaxation des Spinsystems des Kontrastmittels, wodurch die Kontrastwirkung des Kontrastmittels verstärkt wird.

**5.** Verfahren nach Anspruch 2, bei dem der Schritt des Aussetzens dem oszillierenden Magnetfeld in Kombination mit dem stationären Magnetfeld nach dem Schritt des Hydrierens ausgeführt werden soll, und der Schritt des Aussetzens ausgeführt wird zum Verstärken des Grades der Polarisation eines bildgebenden Kerns des Kontrastmittels, wodurch die Kontrastwirkung des Kontrastmittels verstärkt wird.

**6.** Verfahren nach Anspruch 5, bei dem der Schritt des Aussetzens dem oszillierenden Magnetfeld in Kombination mit dem stationären Magnetfeld umfasst Aussetzen des Kontrastmittels mindestens einer Serie von Pulsen des oszillierenden Magnetfelds (rf-Puls).

**7.** Verfahren nach Anspruch 6, bei dem der Schritt des Aussetzens umfasst:

- Anwenden (420) einer ersten Serie von Pulsen der Larmor-Frequenz der bildgebenden Kerne des hydrierten Kontrastmittels und Verzögerungen zwischen den Pulsen, wobei die erste Serie angepasst ist, um das System in einen Zustand zu bringen, der aus einer Quantenkohärenz von 0 besteht, die die Protonen und die Bildgebungskerne beinhaltet;
- Anwenden (430-480) einer zweiten Serie von Pulsen der Larmor-Frequenz der bildgebenden Kerne des hydrierten Kontrastmittels und Verzögerungen zwischen den Pulsen, wobei die zweite Serie angepasst ist, um ein schrittweises Aufbauen der Kohlenstoff-Polarisation in der Richtung der Achse des äußeren Feldes zu ergeben.

**8.** Verfahren nach Anspruch 6, bei dem der Schritt des Aussetzens umfasst:

(a) —Anwenden (420) einer Serie von $180°_x$-Pulsen gefolgt von Verzögerungen ($t_i$) auf die bildgebenden Kerne;
(b) -Anwenden (430) eines $90°_y$-Pulses auf Kohlenstoff;
(c) -Warten (440) für t/2 s;
(d) -gegebenenfalls Anwenden (450) von simultanen $180°_x$-Pulsen auf Wasserstoff und die bildgebenden Kerne, um den Effekt von Feldinhomogenitäten zu kompensieren;
(e) -gegebenenfalls Warten (460) für t/2 s;
(f) -Anwenden (470) eines Pulses mit einem Winkel $\phi_x$ auf die bildgebenden Kerne;
(g) -gegebenenfalls Wiederholen der Schritte c bis f, um ein schrittweises Aufbauen der Polarisation der bildgebenden Kerne in der Richtung der Achse des äußeren Feldes zu erzeugen, wobei der Winkel $\phi_x$ in jeder Wiederholung unterschiedlich sein kann.

**9.** Verfahren nach Anspruch 6, bei dem der Schritt des Aussetzens umfasst:

- Anwenden (520) einer ersten Serie von Pulsen der Larmor-Frequenz auf die bildgebenden Kerne des hydrierten Kontrastmittels und Verzögerungen zwischen den Pulsen, wobei die erste Serie angepasst ist, um das System in einen Zustand zu bringen, der aus einer Quantenkohärenz von 0 besteht, die die Protonen und die bildgebenden Kerne beinhaltet;
- Anwenden (530-540) einer zweiten Serie von Pulsen und Verzögerungen zwischen den Pulsen, die Pulse der Larmor-Frequenz der bildgebenden Kerne des hydrierten Kontrastmittels alternierend mit Pulsen der Larmor-Frequenz des Wasserstoffs des hydrierten , Kontrastmittels umfassen, wobei die zweite Serie angepasst ist, um eine Zwei-Protonen-Doppel-Quantenkohärenz in eine Drei-Spin-Kohärenz umzuwandeln, die die Spins der bildgebenden Kerne beinhaltet;
- Anwenden (570) von simultanen $90°_y$- und $90°_\phi$-Pulsen auf die bildgebenden Kerne bzw. Wasserstoff, angepasst zum Erzeugen einer transversalen Polarisation der bildgebenden Kerne.

**10.** Verfahren nach Anspruch 6, bei dem der Schritt des Aussetzens umfasst:

- Anwenden (520) einer Serie von $180°_x$-Pulsen, gefolgt von Verzögerungen $t_1$ auf die bildgebenden Kerne
- Anwenden (530) eines $90°_y$-Pulses auf Wasserstoff;
- Warten (540) für $t_2/2$ s;
- gegebenenfalls Anwenden (550) von simultanen $180°_x$-Pulsen auf Wasserstoff und die bildgebenden Kerne, um den Effekt von Feldinhomogenitäten zu kompensieren;
- gegebenenfalls Warten (560) für $t_2/2$ s;
- Anwenden von simultanen (570) 90°$_y$- und 90°$_\phi$-Pulsen auf die bildgebenden Kerne und Wasserstoff;
- Warten (580) für $t_3/2$ s;
- Anwenden (585) von simultanen 180°$_x$-Pulsen auf die bildgebenden Kerne und Wasserstoff;
- Warten (590) für $t_3/2$ s;
- Anwenden (595) eines -90°$_y$-Pulses auf Kohlenstoff.

11. Verfahren nach einem der Ansprüche 7 bis 10, bei dem einer oder mehrere der Radiofrequenz-Pulse entweder in der Amplitude, Phase oder Frequenz oder einer beliebigen Kombination davon zusammengesetzt oder moduliert sind.

12. Vorrichtung zum Herstellen eines MR-Kontrastmittels, wobei die Vorrichtung umfasst eine magnetische Behandlungseinheit (240), angepasst zur magnetischen Behandlung des Kontrastmittels,
**dadurch gekennzeichnet, dass** die magnetische Behandlungseinheit (240) Mittel zum Erzeugen eines oszillierenden Magnetfeldes und Mittel zum Erzeugen eines stationären Magnetfeldes umfasst.

13. Vorrichtung nach Anspruch 11, bei der die magnetische Behandlungseinheit (240) mit einem Hydrierreaktor (210) kombiniert ist.

14. Vorrichtung nach Anspruch 12 oder 13, bei der die magnetische Behandlungseinheit (240) im wesentlichen das Magnetsystem eines NMR-Spektrometers umfasst.

15. Vorrichtung nach Anspruch 14, bei der das Magnetsystem eines NMR-Spektrometers zusätzlich zum Analysieren des erzeugten Kontrastmittels mit NMR-Spektroskopie verwendet wird.

16. Vorrichtung nach Anspruch 12 oder 13, bei der die magnetische Behandlungseinheit (240) ein Helmholtz-Paar (360) zum Erzeugen des stationären Magnetfelds und eine NMR-Spule (360) zum Erzeugen des oszillierenden Magnetfelds umfasst.

17. Computerprogrammprodukt, das direkt in den internen Speicher eines Verarbeitungsmittels innerhalb einer Verarbeitungseinheit zum Steuern des Verfahrens und der Vorrichtung zum Herstellen eines MR-Kontrastmittels ladbar ist, umfassend das Softwarecode-Mittel, das zum Steuern der Schritte nach einem der Ansprüche 1 bis 11 angepasst ist.

18. Computerprogrammprodukt, gespeichert auf einem in einem Computer nutzbaren Medium, umfassend ein lesbares Programm, das angepasst ist zum Starten eines Verarbeitungsmittels in einer Verarbeitungseinheit zum Steuern des Verfahrens und der Vorrichtung zum Herstellen eines MR-Kontrastmittels, zum Steuern der Ausführung der Schritte nach einem der Ansprüche 1 bis 11.

**Revendications**

1. Procédé de production d'agent de contraste RM, le procédé comprenant les étapes de :

   obtention (100) d'une solution dans un solvant d'un composé de substrat insaturé, pouvant être hydrogéné et d'un catalyseur d'hydrogénation d'un composé de substrat, dans lequel le composé de substrat comprend des noyaux d'imagerie ;
   hydrogénation (110) du substrat avec du gaz hydrogène ($H_2$) enrichi en para-hydrogène (p-$^1H_2$) afin de former un agent de contraste hydrogéné ;
   exposition (120) de l'agent de contraste à un champ magnétique oscillant en association avec un champ magnétique constant afin de renforcer les effets de contraste de l'agent de contraste adapté à une utilisation dans une application RM.

2. Procédé selon la revendication 1, dans lequel le champ magnétique oscillant oscille avec une fréquence comprise dans l'intervalle des radiofréquences (par exemple, de 10 Hz environ à plusieurs GHz).

3. Procédé selon la revendication 1, dans lequel le champ magnétique oscillant oscille avec une fréquence comprise dans la plage de 5 KHz à 500 MHz.

4. Procédé selon la revendication 2, dans lequel l'étape d'exposition au champ magnétique oscillant en association avec le champ magnétique constant est exécutée au cours de l'étape d'hydrogénation, dans lequel l'étape d'exposition est exécutée afin de réduire la relaxation du système de spin de l'agent de contraste, de telle sorte que les effets de contraste de l'agent de contraste sont renforcés.

5. Procédé selon la revendication 2, dans lequel l'étape d'exposition au champ magnétique oscillant en association avec le champ magnétique constant doit être exécutée après l'étape d'hydrogénation, l'étape d'exposition est exécutée afin d'augmenter le degré de polarisation d'un noyau d'imagerie de l'agent de contraste, de telle sorte que les effets de contraste de l'agent de contraste sont renforcés.

6. Procédé selon la revendication 5, dans lequel l'étape d'exposition au champ magnétique oscillant en association avec le champ magnétique constant comprend l'exposition de l'agent de contraste à au moins une série d'impulsions du champ magnétique oscillant (impulsions de radiofréquence).

7. Procédé selon la revendication 6, dans lequel l'étape d'exposition comprend :

l'application (420) d'une première série d'impulsions à la fréquence de Larmor aux noyaux d'imagerie de l'agent de contraste hydrogéné et de délais entre les impulsions, les premières séries étant adaptées afin d'amener le système dans un état consistant en une cohérence de quantum nulle impliquant les protons et les noyaux d'imagerie ;
l'application (430 à 480) d'une seconde série d'impulsions à la fréquence de Larmor aux noyaux d'imagerie de l'agent de contraste hydrogéné et de délais entre les impulsions, la seconde série étant adaptée afin d'obtenir un établissement progressif de la polarisation du carbone dans la direction de l'axe du champ externe.

8. Procédé selon la revendication 6, dans lequel l'étape d'exposition comprend :

(a) l'application (420) d'une série d'impulsions à $180°_x$ suivies par des délais (ti) sur les noyaux d'imagerie ;
(b) l'application (430) d'une impulsion à $90°_y$ sur du carbone ;
(c) l'attente (440) pendant t/2 s ;
(d) de manière optionnelle, l'application (450) simultanée d'impulsions à $180°_x$ sur l'hydrogène et les noyaux d'imagerie dans le but de corriger l'effet de non homogénéités de champ ;
(e) de manière optionnelle, l'attente (460) pendant t/2 s ;
(f) l'application (466) d'une impulsion avec un angle $\Phi_X$ sur les noyaux d'imagerie ;
(g) en variante, la répétition des étapes (c) à (f) afin d'obtenir un établissement progressif de la polarisation de noyaux d'imagerie dans le sens de l'axe du champ externe, dans lequel l'angle $\Phi_X$ peut être différent à chaque répétition.

9. Procédé selon la revendication 6, dans lequel l'étape d'exposition comprend :

l'application (520) d'une première série d'impulsions à la fréquence de Larmor aux noyaux d'imagerie de l'agent de contraste hydrogéné et de délais entre les impulsions, la première série étant adaptée afin de porter le système dans un état consistant en une cohérence de quantum nulle impliquant les protons et les noyaux d'imagerie ;
l'application (530 à 540) d'une seconde série d'impulsions, et de délais entre les impulsions, comprenant des impulsions à la fréquence de Larmor aux noyaux d'imagerie de l'agent de contraste hydrogéné alternées avec des impulsions à la fréquence de Larmor à l'hydrogène de l'agent de contraste hydrogéné, la seconde série étant adaptée afin de transformer une cohérence à double quantum de deux protons en une cohérence à trois spins impliquant les spins des noyaux d'imagerie ;
l'application (570) simultanée d'impulsions à $90°_y$ et à $90°_\varphi$ respectivement sur les noyaux d'imagerie et l'hydrogène, adaptée afin de produire une polarisation transversale des noyaux d'imagerie.

10. Procédé selon la revendication 6, dans lequel l'étape d'exposition comprend :

l'application (520) d'une série d'impulsions à $180°_x$ suivies par des délais $t_1$ sur les noyaux d'imagerie ;
l'application (530) d'une impulsion à $90°_y$ sur l'hydrogène ;
l'attente (540) pendant $t_2/2$ s ;

de manière optionnelle, l'application (550) simultanée d'impulsions à 180°$_x$ sur l'hydrogène et les noyaux d'imagerie dans le but de corriger l'effet de non homogénéité de champ ;
de manière optionnelle, l'attente (560) pendant $t_2/2$ s ;
l'application simultanée (570) d'impulsions à 90°$_y$ et à 90°$_\Phi$ sur les noyaux d'imagerie et l'hydrogène ;
l'attente (580) pendant $t_3/2$ s ;
l'application (585) simultanée d'impulsions à 180°$_x$ sur les noyaux d'imagerie et l'hydrogène ;
l'attente (590) pendant $t_3/2$ s ;
l'application (595) d'une impulsions à -90°y sur le carbone.

**11.** Procédé selon l'une quelconque des revendications 7 à 10, dans lequel une ou plusieurs des impulsions de radiofréquence sont soit composites soit modulées en amplitude, en phase ou en fréquence soit une combinaison quelconque de celles-ci.

**12.** Dispositif destiné à produire un agent de contraste RM, le dispositif comprenant une unité de traitement magnétique (240) adaptée afin d'assurer le traitement magnétique de l'agent de contraste, **caractérisé en ce que** l'unité de traitement magnétique (240) comprend un moyen destiné à produire un champ magnétique oscillant et un moyen destiné à produire un champ magnétique constant.

**13.** Dispositif selon la revendication 11, dans lequel ladite unité de traitement magnétique (240) est associée à un réacteur d'hydrogénation (210).

**14.** Dispositif selon la revendication 12 ou 13, dans lequel ladite unité de traitement magnétique (240) comprend principalement le système magnétique d'un spectromètre RMN.

**15.** Dispositif selon la revendication 14, dans lequel ledit système magnétique d'un spectromètre RMN est, en outre, utilisé afin d'analyser, par spectroscopie RMN, l'agent de contraste produit.

**16.** Dispositif selon la revendication 12 ou 13, dans lequel ladite unité de traitement magnétique (240) comprend une paire Helmholtz (360) afin de produire le champ magnétique constant et une bobine RMN (360) afin de produire le champ magnétique oscillant.

**17.** Produit formant programme informatique pouvant être chargé directement dans la mémoire interne d'un moyen de traitement à l'intérieur d'une unité de traitement destinée à commander le procédé et le dispositif de production d'agent de contraste RM, comprenant les moyens formant code logiciel adaptés afin de commander les étapes de l'une quelconque des revendications 1 à 11.

**18.** Produit formant programme informatique mémorisé sur un milieu pouvant être utilisé par un ordinateur, comprenant un programme pouvant être lu, adapté afin d'amener un moyen de traitement, dans une unité de traitement destinée à commander le procédé et le dispositif de production d'agent de contraste RM, à commander une exécution des étapes selon l'une quelconque des revendications 1 à 11.

100

110

120

*FIG. 1*

FIG. 2

FIG. 3

*FIG. 4*

```
┌─────────────────────────┐
│           500           │
└─────────────────────────┘
             │
             ▼
     ┌───────────────┐
     │      510      │
     └───────────────┘
             │
             ▼
┌─────────────────────────────┐
│             520             │
└─────────────────────────────┘
             │
             ▼
┌──────────────────────────────┐
│             530              │
└──────────────────────────────┘
             │
             ▼
     ┌───────────────┐
     │      540      │
     └───────────────┘
             │
             ▼
┌──────────────────────────────┐
│             550              │
└──────────────────────────────┘
             │
             ▼
     ┌───────────────┐
     │      560      │
     └───────────────┘
             │
             ▼
┌────────────────────────────────┐
│              570               │
└────────────────────────────────┘
             │
             ▼
     ┌───────────────┐
     │      580      │
     └───────────────┘
             │
             ▼
┌──────────────────────────────┐
│             585              │
└──────────────────────────────┘
             │
             ▼
     ┌───────────────┐
     │      590      │
     └───────────────┘
             │
             ▼
┌──────────────────────────────┐
│             595              │
└──────────────────────────────┘
```

*FIG. 5*